# EUROPEAN PATENT APPLICATION

(11) **EP 3 090 984 A1**
(43) Date of publication of application: **09.11.2016**
(21) Application number: 15166829.0
(22) Date of filing: 07.05.2015
(51) Int. Cl.: C01B 21/086, C01B 21/092, C01B 21/093, C07C 303/40, C07C 311/48, H01M 10/052, H01M 10/0568

(54) **METHOD FOR PREPARATION OF THE SALTS OF CERTAIN AMINES AND BIS(FLUOROSULFONYL)-IMIDE**

(71) Applicant: Lonza Ltd, 3930 Visp (CH)
(72) Inventor: BERSIER, Michael, 3938 Ausserberg (CH)

(57) **Abstract**

The invention discloses a method for the preparation of the salts of certain amines and bis(fluorosulfonyl)-imide starting from bis(chlorosulfonyl)-imide and a mixture of HF with said certain amines.

## Description

The invention discloses a method for the preparation of the salts of certain amines and bis(fluorosulfonyl)-imide starting from bis(chlorosulfonyl)-imide and a mixture of HF with said certain amines.

### BACKGROUND OF THE INVENTION

In the following text,
ClSI bis(chlorosulfonyl)-imide, that is compound of formula (2);
CSI chlorosulfonyl isocyanate, that is compound of formula (1);
FSI bis(fluorosulfonyl)-imide, that is compound of formula (5); 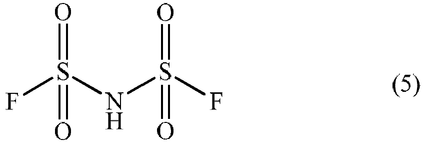
MEP 2-methyl-5-ethyl-pyridine, that is compound of formula (3);
MEP-FSI the salt of MEP and FSI, that is compound of formula (4); 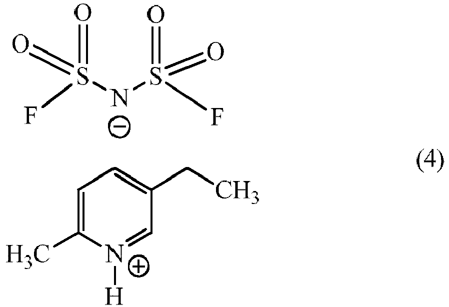
ambient pressure maens usually 1 bar, depending on the weather;
halide means F , Cl⁻, Br or I⁻, preferably Cl⁻, Br or I⁻, more preferably Cl⁻ or Br ; halogen means F, Cl, Br or I, preferably Cl, Br or I, more preferably Cl or Br; if not otherwise stated.
FSI is an intermediate used for the production of electrolytes in electrochemical devices such as in lithium ion batteries in form of its lithium salt LiFSI.
WO 2009/123328 A1 discloses a method for preparation of metal salts of FSI starting from ClSI, these metal salts are then converted in a second step to salts of various amines and FSI in a cation exchange reaction.
WO 2015/012897 A1 discloses a method for producing FSI from ClSI using HF, wherein the HCl that is produced in the reaction is selectively removed. This requirement results in a complex apparatus set up.
WO 2014/080120 A1 discloses a method for the preparation of FSI from ClSI, wherein the reaction is done in an organic solvent.

There was a need for a method for preparation of MEP-FSI that allows an uncomplicated apparatus set up compared to the prior art, that does not use a solvent, and that has few steps.

Unexpectedly a method for preparation of MEP-FSI was found starting from ClSI, does not need a solvent, which can be done in a relatively uncomplicated apparatus set up, and that has few steps. The amine salts of FSI can be prepared directly from ClSI.

### SUMMARY OF THE INVENTION

Subject of the invention is a method for the preparation of compound of formula (IV); 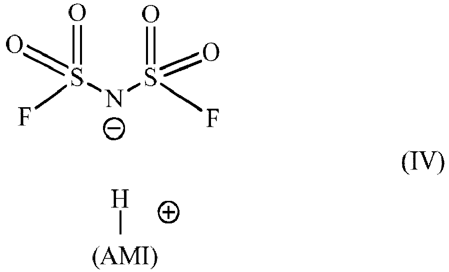 AMI is selected from the group consisting of pyridine, 2-methylpyridine, 3-methylpyridine, 4-methylpyridine, compound of formula (3) and triethylamine; 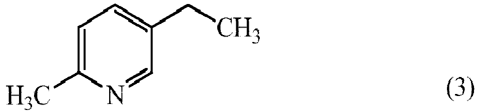 the method comprises a step ST2;
ST2 comprises a reaction REAC2, in REAC2 a mixture MIXTURE 1 is reacted with a mixture MIXTURE-TRIPLE;
MIXTURE-TRIPLE comprises three components, a compound of formula (2), a compound of formula (1) and chlorosulfonic acid, in the relative ratio of from
2 to 100 % of compound of formula (2),
49 to 0 % of compound of formula (1), and
49 to 0 % of chlorosulfonic acid;
the % are % by weight and are based on the combined weight of the three components in MIXTURE-TRIPLE; the relative ratios of the three components add up to 100%; 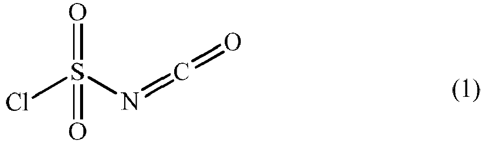 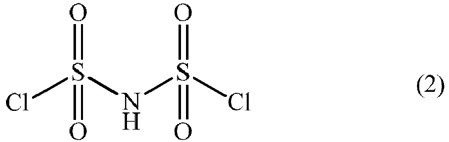 MIXTURE 1 is a mixture of HF with AMI;
REAC2 results in a reaction mixture REACMIXTURE2.

### DETAILED DESCRIPTION OF THE INVENTION

Preferably, the method comprises a second step, a step ST1, ST1 is done before ST2; ST1 comprises the preparation of MIXTURE1 by a mixing MIXING1 of HF with AMI.

Preferably, AMI is compound of formula (3).

MIXTURE-TRIPLE is commercially available and can be prepared in according to known methods, such as disclosed in WO 2009/123328 A1, e.g. in Synthesis Example 2, or according to the method disclosed in WO 2015/004220 A1.

Preferably, the total content of the three components in MIXTURE-TRIPLE is of from 50 to 100%, more preferably of form 75 to 100%, even more preferably of from 90 to 100%, especially of from 95 to 100%, more especially of from 97.5 to 100%, even more especially of from 98 to 100%, the % being % by weight based on the total weight of MIXTURE-TRIPLE.

In one preferred embodiment, MIXTURE-TRIPLE is compound of formula (2).

In another preferred embodiment, MIXTURE-TRIPLE consists essentially of the three components compound of formula (2), compound of formula (3), and chlorosulfonic acid.

In another preferred embodiment, the relative ratio of the three components in MIXTURE-TRIPLE is of from
2 to 98 % of compound of formula (2),
49 to 1 % of compound of formula (1), and
49 to 1 % of chlorosulfonic acid;
more preferably of form
2 to 96 % of compound of formula (2),
49 to 2 % of compound of formula (1), and
49 to 2 % of chlorosulfonic acid;
even more preferably of from
50 to 96 % of compound of formula (2),
25 to 2 % of compound of formula (1), and
25 to 2 % of chlorosulfonic acid;
especially of from
70 to 96 % of compound of formula (2),
15 to 2 % of compound of formula (1), and
15 to 2 % of chlorosulfonic acid;
more especially of from
75 to 96 % of compound of formula (2),
12.5 to 2 % of compound of formula (1), and
12.5 to 2 % of chlorosulfonic acid;
the % are % by weight based on the combined weight of the three components in MIXTURE-TRIPLE; the relative ratios of the three components add up to 100%.

For the purpose of this invention, the molar content of a sample of MIXTURE-TRIPLE is assumed to be identical to the molar content of a sample of a pure compound of formula (2) of the same weight.

Preferably, the molar amount of HF is from 2 to 40 times, more preferably from 2 to 20 times, and even more preferably from 2 to 12.5 times, especially from 2 to 5 times, more especially from 2 to 3.5 times, based on the molar amount of MIXTURE-TRIPLE.

Preferably, the molar amount of AMI is of from 0.75 to 10 times, more preferably of from 0.8 to 5 times, and even more preferably of from 0.8 to 2.5 times, especially of from 0.8 to 1.5 times, more especially of from 0.8 to 1.2 times, even more especially of from 0.9 to 1.1 times, based on the molar amount of MIXTURE-TRIPLE.

Preferably, MIXING1 is done at a temperature of from -80 °C to 50 °C, more preferably from -30 °C to 40 °C, even more preferably from -25 °C to 20 °C.

Preferably, MIXING1 is done at a pressure of from ambient pressure to 10 bar, more preferably from ambient pressure to 5 bar, even more preferably from ambient pressure to 2 bar.

Preferably, the time of MIXING 1 is of from 5 min to 10 h, more preferably of from 5 min to 7.5 h, even more preferably of from 5 min to 5 h.

Preferably, REAC2 is done at a temperature of from -40 °C to 200 °C, more preferably from -30 °C to 175 °C, even more preferably from -20 °C to 160 °C.

In another preferred embodiments, REAC2 is done at a temperature of from -40 °C to 200°C, from -40 °C to 175°C, from -40 °C to 160°C, from -40 °C to 150°C, more preferably from -30 °C to 125 °C, even more preferably from -20 °C to 100 °C, especially -20 °C to 75 °C.

Preferably, REAC2 is done at a pressure of from ambient pressure to 10 bar, more preferably from ambient pressure to 5 bar, even more preferably from ambient pressure to 2 bar.

Preferably, the time of REAC2 is from 1 h to 48 h, more preferably from 2 h to 24 h, even more preferably from 5 h to 18 h.

MIXING1 and/or REAC2 can be done in a discontinuous way, that is batch wise, or in a continuous way, that is in form of a continuous reaction.

During REAC2 HCl is formed.

Preferably, HCl is not removed during REAC2 from the reaction mixture.

HF can be used stoichiometrically or in excess. In any of these two cases unreacted HF can be present in REACMIXTURE2 after REAC2.

Preferably, the method comprises a third step ST3, step ST3 is done after step ST2, step ST3 comprises a recovery RECOV3 of any unreacted HF.

Preferably, RECOV3 is done by a purging PURG3 of REACMIXTURE2 with N₂.

Preferably, PURG3 is done with a flow rate of from 1 to 150 l/min, more preferably of from 10 to 100 l/min, even more preferably of from 20 to 90 l/min.

Preferably, PURG3 is done at a temperature of from 15 °C to 150 °C, more preferably from 15 °C to 125 °C, even more preferably from 15 °C to 100 °C.

Preferably, the time of PURG3 is from 10 min to 5 h, more preferably from 30 min to 3 h.

In another preferred embodiment, RECOV3 is done under a vacuum VAC3, which is applied to REACMIXTURE2.

Preferably, VAC3 is applied at a temperature of from 15 °C to 150 °C, more preferably from 15 °C to 125 °C, even more preferably from 15 °C to 100 °C.

Preferably, the time where VAC3 is applied is from 10 min to 5 h, more preferably from 30 min to 3 h.

Preferably, the pressure of VAC3 is of from 10 to 500 mbar, more preferably of from 10 to 250 mbar, even more preferably of from 10 to 150 mbar.

In another preferred embodiment, RECOV3 is done by PURG3 and by applying VAC3. PURG3 can be done before, during or after applying VAC3.

Compound of formula (IV) can be used as such after its preparation.

### Examples

### Methods

¹⁹F-NMR was done in CD₃CN with benzenesulfonyl fluoride as internal standard; the yield of MEP-FSI is given based on the molar amount of compound of formula (2) or of MIXTURE-TRIPLE, respectively;
if not otherwise stated.

### Materials

A mixture MIXTURE-TRIPLE-90-5-5 is prepared according to example 15 of WO2015/004220 A1. The conversion of 95% stated in this example 15 of WO2015/004220 A1 means that 5% residual CSI are present in the mixture. It is assumed that therefore the equivalent amount of chlorosulfonic acid is present in the mixture as well. Thereby MIXTURE-TRIPLE-90-5-5 contains ca. 90% of compound of formula (2), 5% of compound of formula (1) and 5% of chlorosulfonic acid, the % being % by weight based on the total weight of MIXTURE-TRIPLE-90-5-5.

### Example 1

18.70 g of anhydrous HF (0.934 mol, 10.0 eq.) were added at -10°C to 11.32 g of MEP (0.0934 mol, 1.0 eq.). The reaction mixture was stirred for 10 min at -10°C. Then 20 g of MIXTURE-TRIPLE-90-5-5 (0.0934 mol, 1.0 eq.) was added dropwise at -10°C. Then the reaction mixture was warmed to 20°C within 2 h and then stirred at 20°C for 12 h. Then the reaction mixture was purged with N₂ (40 l/min) for 1 h at 20°C. 29.72 g of MEP-FSI were obtained as a brown oil (¹⁹F-NMR Yield: 76.1%).

### Example 2

5.61 g of anhydrous HF (0.28 mol, 4.0 eq.) were added at -10°C to 8.49 g of MEP (0.07 mol, 1.0 eq.). The reaction mixture was stirred for 10 min at -10°C. Then 15 g of MIXTURE-TRIPLE-90-5-5 (0.07 mol, 1.0 eq.) was added dropwise at -10°C. Then the reaction mixture was warmed to 20°C within 2 h and then stirred at 20°C for 12 h. Then the reaction mixture was purged with N₂ (40 l/min) for 1 h at 20°C. 22.83 g of MEP-FSI were obtained as a brown oil (¹⁹F-NMR Yield: 75.3%).

### Example 3

4.11 g of anhydrous HF (0.2055 mol, 2.2 eq.) were added at -10°C to 11.32 g of MEP (0.0934 mol, 1.0 eq.). The reaction mixture was stirred for 10 min at -10°C. Then 20 g of MIXTURE-TRIPLE-90-5-5 (0.0934 mol, 1.0 eq.) was added dropwise at -10°C. Then the reaction mixture was warmed to 20°C within 2 h and then stirred at 20°C for 12 h. Then the reaction mixture was purged with N₂ (40 l/min) for 1 h at 20°C. 29.72 g of MEP-FSI were obtained as a brown oil (¹⁹F-NMR Yield: 51.8%).

### Example 4

4.11 g of anhydrous HF (0.2055 mol, 2.2 eq.) were added at -10°C to 11.32 g of MEP (0.0934 mol, 1.0 eq.). The reaction mixture was stirred for 10 min at -10°C. Then 20 g of MIXTURE-TRIPLE-90-5-5 (0.0934 mol, 1.0 eq.) was added dropwise at -10°C. Then the reaction mixture was warmed to 50°C within 2 h and then stirred at 50°C for 12 h. Then the reaction mixture was cooled to 20°C and purged with N₂ (40 l/min) for 1 h at 20°C. 29.72 g of MEP-FSI were obtained as a brown oil (¹⁹F-NMR Yield: 76.6%).

### Example 5

Example 4 was repeated with the sole difference, that 7.40 g of pyridine (0.0934 mol, 1.0 eq.) were used and not 11.32 g of MEP (0.0934 mol, 1.0 eq.).

22.36 g of the salt of pyridine with FSI were obtained as a brown oil (19F-NMR Yield: 70.3%).

### Example 6

Example 4 was repeated with the two differences:
- 9.50 g of triethylamine (0.0934 mol, 1.0 eq.) were used and not 11.32 g of MEP (0.0934 mol, 1.0 eq.).
- The reaction mixture was warmed to 100°C within 2 h and then stirred at 100°C for 12 h, and not 50°C.

23.07 g of the salt of triethylamine with FSI were obtained as a brown oil (¹⁹F-NMR Yield: 67.3%).

### Example 7

Example 4 was repeated with the sole difference, that 8.70 g of 2-picoline (0.0934 mol, 1.0 eq.) were used and not 11.32 g of MEP (0.0934 mol, 1.0 eq.).

26.71 g of the salt of 2-picoline with FSI were obtained as a brown oil (19F-NMR Yield: 72.5%).

### Example 8

Example 4 was repeated with the sole difference, that 8.70 g of 3-picoline (0.0934 mol, 1.0 eq.) were used and not 11.32 g of MEP (0.0934 mol, 1.0 eq.).

29.41 g of the salt of 3-picoline with FSI were obtained as a brown oil (19F-NMR Yield: 77.1%).

### Example 9

Example 4 was repeated with the sole difference, that 8.70 g of 4-picoline (0.0934 mol, 1.0 eq.) were used and not 11.32 g of MEP (0.0934 mol, 1.0 eq.).

25.51 g of the salt of 4-picoline with FSI were obtained as a brown oil (19F-NMR Yield: 77.7%).

## Claims

1. Method for the preparation of compound of formula (IV); 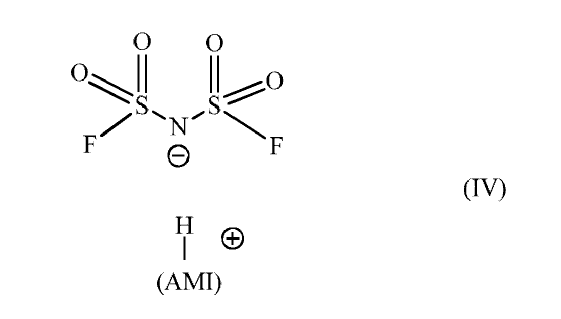 AMI is selected from the group consisting of pyridine, 2-methylpyridine, 3-methylpyridine, 4-methylpyridine, compound of formula (3) and triethylamine; 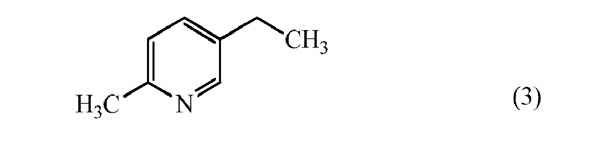 the method comprises a step ST2;
ST2 comprises a reaction REAC2, in REAC2 a mixture MIXTURE 1 is reacted with a mixture MIXTURE-TRIPLE;
MIXTURE-TRIPLE comprises three components, a compound of formula (2), a compound of formula (1) and chlorosulfonic acid, in the relative ratio of from
2 to 100 % of compound of formula (2),
49 to 0 % of compound of formula (1), and
49 to 0 % of chlorosulfonic acid;
the % are % by weight and are based on the combined weight of the three components in MIXTURE-TRIPLE; the relative ratios of the three components add up to 100%; 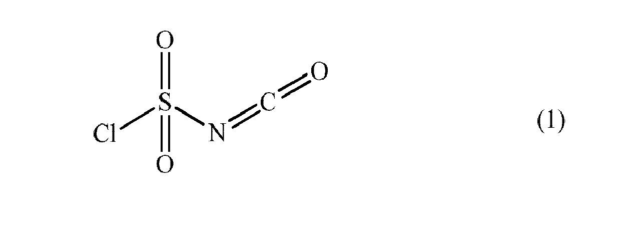 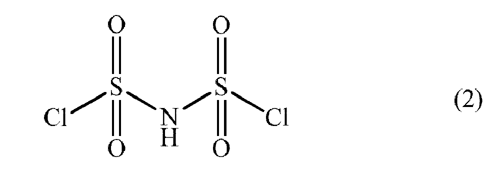 MIXTURE 1 is a mixture of HF with AMI;
REAC2 results in a reaction mixture REACMIXTURE2.

2. Method according to claim 1, wherein
Preferably, the method comprises a second step, a step ST1, ST1 is done before ST2;
ST1 comprises the preparation of MIXTURE1 by a mixing MIXING1 of HF with AMI.

3. Method according to claim 1 or 2, wherein
AMI is compound of formula (3).

4. Method according to one or more of claims 1 to 3, wherein
the total content of the three components in MIXTURE-TRIPLE is of from 90 to 100%, the % being % by weight based on the total weight of MIXTURE-TRIPLE.

5. Method according to one or more of claims 1 to 4, wherein
MIXTURE-TRIPLE is compound of formula (2).

6. Method according to one or more of claims 1 to 4, wherein
the relative ratio of the three components in MIXTURE-TRIPLE is of from 2 to 98 % of compound of formula (2),
49 to 1 % of compound of formula (1), and
49 to 1 % of chlorosulfonic acid;
the % are % by weight based on the combined weight of the three components in MIXTURE-TRIPLE; the relative ratios of the three components add up to 100%.

7. Method according to one or more of claims 1 to 6, wherein
the molar amount of HF is from 2 to 40 times, based on the molar amount of MIXTURE-TRIPLE.

8. Method according to one or more of claims 1 to 7, wherein
the molar amount of AMI is of from 0.75 to 10 times, based on the molar amount of MIXTURE-TRIPLE.

9. Method according to one or more of claims 1 to 8, wherein
REAC2 is done at a temperature of from -40 °C to 150°C.

10. Method according to one or more of claims 1 to 9, wherein
the time of REAC2 is from 1 h to 48 h.

11. Method according to one or more of claims 1 to 10, wherein the method comprises a third step ST3, step ST3 is done after step ST2, step ST3 comprises a
recovery RECOV3 of any unreacted HF.

12. Method according to claim 11, wherein
RECOV3 is done by a purging PURG3 of REACMIXTURE2 with N₂.

13. Method according to claim 11 or 12, wherein
RECOV3 is done under a vacuum VAC3, which is applied to REACMIXTURE2.
